(19) Europäisches Patentamt
European Patent Office
Office européen des brevets

(11) **EP 3 535 582 B1**

(12) **FASCICULE DE BREVET EUROPEEN**

(45) Date de publication et mention
de la délivrance du brevet:
**07.06.2023 Bulletin 2023/23**

(21) Numéro de dépôt: **17803818.8**

(22) Date de dépôt: **06.11.2017**

(51) Classification Internationale des Brevets (IPC):
**G01N 33/49** (2006.01)

(52) Classification Coopérative des Brevets (CPC):
**G01N 33/4905**

(86) Numéro de dépôt international:
**PCT/EP2017/078372**

(87) Numéro de publication internationale:
**WO 2018/083315 (11.05.2018 Gazette 2018/19)**

(54) **PROCEDE DE CARACTERISATION D'UN ECHANTILLON SANGUIN**

VERFAHREN ZUR CHARAKTERISIERUNG EINER BLUTPROBE

PROCESS FOR CHARACTERIZING A BLOOD SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.11.2016 FR 1660749**

(43) Date de publication de la demande:
**11.09.2019 Bulletin 2019/37**

(73) Titulaires:
• **Sorbonne Université**
**75006 Paris 6 (FR)**
• **INSERM (Institut National de la Santé et de la Recherche Médicale)**
**75013 Paris (FR)**
• **Conception De Systemes Et Technologie Mecanique :**
**31320 Castanet Tolosan (FR)**

(72) Inventeurs:
• **RENDU, Francine**
**75014 Paris (FR)**
• **DUFILHO, Monique**
**75015 Paris (FR)**
• **DUGUE, François**
**31450 Pompertuzat (FR)**

(74) Mandataire: **IPAZ**
**Bâtiment Platon**
**Parc Les Algorithmes**
**91190 Saint-Aubin (FR)**

(56) Documents cités:
**EP-A1- 2 053 387       US-A1- 2010 181 210**
**US-A1- 2013 083 311**

## Description

### Domaine technique

La présente invention concerne un procédé de caractérisation d'un échantillon sanguin.

Un tel dispositif permet par exemple à un utilisateur de mesurer et/ou caractériser l'agrégation de plaquettes d'un échantillon sanguin.

### Etat de la technique antérieure

On connaît un procédé de caractérisation d'un échantillon sanguin utilisant un dispositif tel que décrit dans le document WO 2009/053841.

Ce document propose comme procédé de pré-remplir un capillaire avec de l'eau puis d'introduire dans le capillaire un échantillon sanguin. Le passage du front séparant les deux liquides produit une variation de capacitance entre deux électrodes de mesure, qui est mesurée et permet de détecter et/ou caractériser l'état d'activation des plaquettes de l'échantillon sanguin.

Un tel procédé pose cependant deux problèmes techniques correspondant à:

- une amélioration possible de la reproductibilité des mesures, et/ou
- une meilleure discrimination de différents échantillons sanguins,
- une amélioration de la reproduction des conditions physiopathologiques.

Le but de la présente invention est de résoudre au moins un de ces problèmes.

### Exposé de l'invention

Cet objectif est atteint avec un procédé selon la revendication 1.

La solution a de préférence, lors du passage du front par l'entrée du canal, un taux de cisaillement aux parois internes du canal supérieur à 10 000 s$^{-1}$.

La solution a de préférence, pendant la progression du front de l'entrée vers la sortie du canal, un taux de cisaillement aux parois internes du canal qui diminue avec le temps au fur et à mesure de l'avancée du front entre la solution et le gaz, de préférence jusqu'à ce que le front atteigne la sortie du canal.

La solution a de préférence, lors du passage du front par la sortie du canal, un taux de cisaillement aux parois internes du canal inférieur à 1000 s$^{-1}$.

La différence de pression entre l'entrée et la sortie du canal ou une consigne de cette différence de pression est constante pendant la progression de la solution dans le canal, de préférence au moins jusqu'à ce que le front atteigne la sortie du canal et/ou jusqu'à la mesure.

Le canal a de préférence une section d'aire constante de son entrée à sa sortie.

La différence de pression entre l'entrée et la sortie du canal est de préférence créée par aspiration du côté de la sortie du canal.

Le front a de préférence une vitesse de progression dans le canal qui diminue avec le temps pendant la progression de la solution dans le canal, de préférence jusqu'à ce que le front atteigne la sortie du canal.

Le gaz est de préférence de l'air.

La mesure du signal électrique est de préférence effectuée alors que :

- la progression de la solution de l'entrée vers la sortie du canal est dans un régime stationnaire, et/ou
- le canal est rempli par la solution de l'entrée jusqu'à la sortie du canal.

Les électrodes sont de préférence situées dans une deuxième moitié du canal à partir de l'entrée du canal. Le canal a de préférence une forme de serpentin avec plusieurs boucles. Les électrodes de la zone de mesure sont de préférence situées sur la dernière boucle du canal (par rapport à l'entrée du canal).

Le procédé selon l'invention peut comprendre en outre, à partir de la mesure du signal électrique, un calcul d'une donnée de discrimination plaquettaire et/ou de coagulation.

Les moyens de mesure, de préférence, imposent une intensité électrique entre les électrodes et mesurent une tension entre les électrodes pendant que l'intensité électrique est imposée, cette intensité électrique étant de préférence constante pendant la mesure.

Le procédé selon l'invention peut comprendre une intégration, dans le temps, de la tension mesurée.

Le procédé selon l'invention peut comprendre :

- une détection, de préférence par des photodiodes, du passage du front entre les électrodes et la sortie du canal, et/ou
- une mesure, de préférence par des photodiodes, d'une vitesse de la solution dans le canal, et un calcul, par des moyens techniques de traitement, d'une viscosité de la solution en fonction de la vitesse mesurée.

Le procédé selon l'invention peut comprendre une stabilisation en température du canal, de préférence à une température comprise entre 36°C et 38 °C.

La solution est de préférence un échantillon sanguin provenant d'un prélèvement sur un homme ou un animal.

Le procédé selon l'invention ne comprend de préférence pas d'ajout d'activateur plaquettaire et/ou de coagulation à l'échantillon sanguin tel que prélevé.

**Description des figures et modes de réalisation**

D'autres avantages et particularités de l'invention apparaîtront à la lecture de la description détaillée de mises en oeuvre et de modes de réalisation nullement limitatifs, et des dessins annexés suivants :

- la figure 1 est une vue schématique de dispositif 1 utilisé pour mettre en oeuvre un mode de réalisation de procédé selon l'invention, ledit dispositif 1 comprenant une cartouche 5 et des moyens de mesure 10,
- la figure 2 est une vue en perspective de la cartouche 5 du dispositif 1, cette cartouche 5 comprenant un canal 2 prévu pour se remplir d'une solution,
- la figure 3 illustre la variation du taux de cisaillement de la solution sur des parois internes du canal 2, en fonction de la distance entre l'entrée 3 du canal 2 et le front solution/gaz,
- la figure 4 illustre la variation de la vitesse du front solution/gaz, en fonction de la distance entre l'entrée 3 du canal 2 et le front solution/gaz,
- la figure 5 est un schéma d'une carte électronique des moyens de mesure 10, et
- la figure 6 illustre différentes valeurs moyennes de la donnée de discrimination plaquettaire (intégration du signal de tension) pour différentes populations (sujets sains, patients traités par monothérapies antiagrégantes plaquettaires et patients traités par bithérapies antiagrégantes plaquettaires).

Ces modes de réalisation n'étant nullement limitatifs, on pourra notamment considérer des variantes de l'invention ne comprenant qu'une sélection de caractéristiques décrites ou illustrées par la suite isolées des autres caractéristiques décrites ou illustrées (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique de préférence fonctionnelle sans détails structurels, et/ou avec seulement une partie des détails structurels si cette partie uniquement est suffisante pour conférer un avantage technique ou à différencier l'invention par rapport à l'état de la technique antérieure.

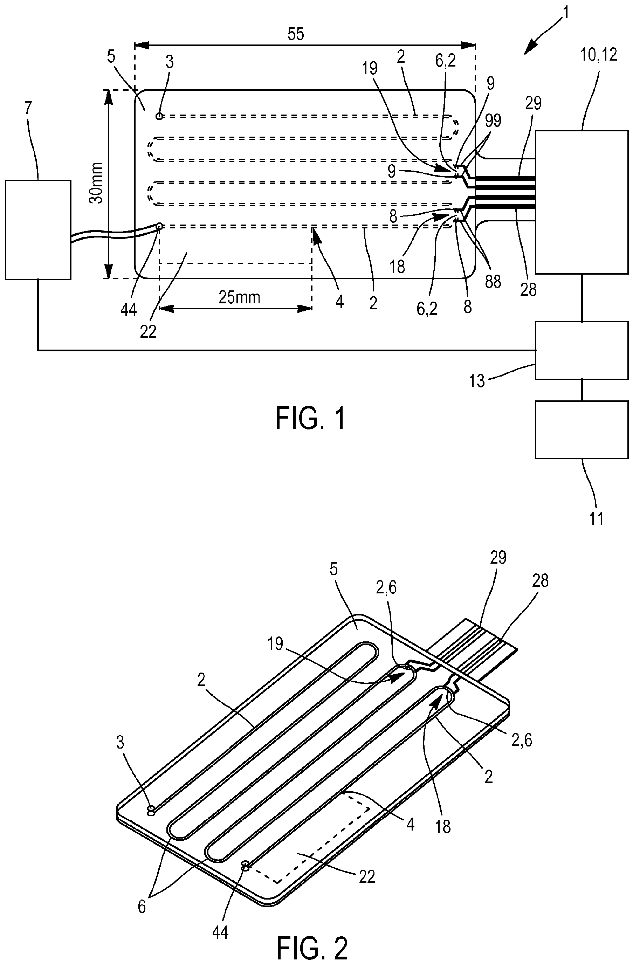

On va tout d'abord décrire, en référence aux figures 1 à 6, un mode de réalisation préférentiel de procédé selon l'invention.

Le type de dispositif 1 utilisé pour mettre en oeuvre le mode de réalisation de procédé selon l'invention correspond au type général de dispositif tel que décrit dans la demande WO2009/053841.

Ce mode de réalisation de procédé selon l'invention comprend une réalisation, dans un canal 2 du dispositif utilisé 1, d'un vide primaire (typiquement d'environ 5 kPa par rapport à la pression atmosphérique ou à la pression environnant le dispositif utilisé).

Ce mode de réalisation de procédé selon l'invention de caractérisation d'un échantillon sanguin, comprend une insertion, dans le canal 2 (encore soumis au vide primaire), d'une solution comprenant des plaquettes sanguines (typiquement de 30 $\mu$l à 200 $\mu$l de solution) et des facteurs de coagulation.

Le canal 2 comprend une entrée 3 (consistant typiquement en un perçage de 1 mm de diamètre) et une sortie 4, la solution étant insérée par l'entrée 3 du canal 2.

La sortie 4 du canal débouche sur un réservoir 22, qui sert à stocker la solution sanguine afin que la solution sanguine ne s'échappe pas de la cartouche 5 pour des raisons d'hygiène et de sécurité.

Le canal 2 est plus précisément un capillaire.

La solution est une solution de sang total non dilué comprenant des plaquettes sanguines.

La solution est un échantillon sanguin provenant d'un prélèvement réalisé sur un homme ou un animal, le procédé ne comprenant pas d'ajout d'activateur plaquettaire (tel que l'adénosine diphosphate (ADP), d'acide arachidonique, de collagène, de sérotonine, de thrombine, etc.) et/ou d'activateur de coagulation (tel que du facteur tissulaire, le facteur XII, la thrombine, etc.) à l'échantillon sanguin tel que prélevé.

Le canal 2 est réalisé dans une cartouche 5 (qui peut être monobloc ou résulter d'une superposition de plusieurs plaques), de préférence dans un matériau plastique (typiquement du polytéréphtalate d'éthylène, et/ou du polycarbonate).

Par exemple, la cartouche 5 est un assemblage :

- d'une plaque supérieure en polycarbonate transparent pour suivre la circulation de la solution dans le canal 2 et le réservoir 22 qui sont usinés sur une des faces de cette plaque supérieure, et
- d'une plaque inférieure en PC Xantar 3730 LDS Noir, un polycarbonate contenant des particules de cuivre activables par laser pour permettre le dépôt électrolytique des électrodes décrites par la suite,

ces deux plaques étant scellées hermétiquement ensemble (par exemple par un joint, et/ou par collage et/ou par soudure laser, etc.) pour maintenir l'étanchéité du canal 2 et supporter des pressions de 5 à 10 kPa.

Le canal 2 a une longueur (entre son entrée 3 et sa sortie 4) de 260 mm.

Le canal 2 a une forme de serpentin avec plusieurs boucles 6, typiquement trois à dix-neuf boucles

Le canal 2 a une aire de section interne qui est constante de son entrée 3 à sa sortie 4.

Le canal 2 a une section d'aire constante de son entrée 3 à sa sortie 4, typiquement de forme carrée, rectangulaire ou ronde ou ovale. Sa section a un diamètre et/ou une largeur et/ou une hauteur typiquement supérieur à 50 $\mu$m et/ou inférieur à 900 $\mu$m.

Dans cet exemple particulier, le canal 2 à une section rectangulaire de 320 $\mu$m de hauteur sur 350 $\mu$m de largeur.

Ce mode de réalisation de procédé selon l'invention comprend en outre une création d'une différence de pression entre l'entrée 3 et la sortie 4 du canal 2, de manière à faire progresser la solution de l'entrée 3 vers la sortie 4 du canal 2.

La différence de pression entre l'entrée 3 et la sortie 4 du canal 2 est créée par aspiration du côté de la sortie 4 du canal 2.

Les moyens 7 utilisés pour créer cette différence de pression assurent une évolution de la vitesse du front solution/gaz dans le canal 2 par régulation de la dépression $\Delta$P en aval à la sortie 4, la pression amont à l'entrée 3 étant la pression atmosphérique. Les moyens 7 comprennent par exemple une pompe à vide (de référence KNF NMP05M de 6 VDC) et deux électrovannes (LFVA_50210H THE LEE CO, ESSEX). La pression limite que peut fournir le système est de 70 kPa absolu environ (-30 kPa par rapport à la pression atmosphérique). La régulation de la pression d'aspiration est précise à 0,5 kPa autour de seuil de consigne de pression fixé pour avoir une pression différentielle de 5 kPa par rapport à la pression atmosphérique du jour de la mesure.

Le réservoir 22 est relié d'un côté à la sortie 4 et d'un autre côté à un passage 44 (qui est typiquement un trou ou canal) de communication avec les moyens 7.

Le dispositif 1 comprend, de manière optionnelle, des moyens de détection « de mesure ». Ces moyens de détection comprennent par exemple une photodiode, et sont disposés dans ou orientés vers :

- la sortie 4 du canal 2, ou
- le réservoir 22, en étant plus proche de la sortie 4 que du passage 44.

Ces moyens de détection « de mesure » sont :

- agencés pour détecter un niveau déterminé de remplissage du réservoir 22 par la solution sanguine et/ou une arrivée de la solution sanguine par la sortie 4 du canal 2, et
- reliés à des moyens de mesure 10 d'un signal électrique qui sont déclenchés lorsque l'on détecte ce niveau déterminé de remplissage du réservoir 22 par la solution sanguine (par exemple 1/5ème de remplissage) et/ou une arrivée de la solution sanguine par la sortie 4.

Ainsi, dans le procédé selon l'invention, on déclenche la mesure (par les moyens de mesure 10) d'un signal électrique après (de préférence juste après) avoir détecté ce niveau déterminé de remplissage du réservoir 22 par la solution sanguine et/ou une arrivée de la solution sanguine par la sortie 4 du canal 2.

Le dispositif 1 comprend, de manière optionnelle, des moyens de détection « d'arrêt ». Ces moyens de détection comprennent par exemple une photodiode, et sont disposés dans ou orientés vers :

- le passage 44, ou
- le réservoir 22, en étant plus proche du passage 44 que de la sortie 4.

Ces moyens de détection « d'arrêt » sont :

- agencés pour détecter un niveau déterminé de remplissage du réservoir 22 par la solution sanguine et/ou une arrivée de la solution sanguine par le passage 44, et

EP 3 535 582 B1

- reliés à des moyens (la carte 13) qui commandent les moyens 7 utilisés pour créer la différence de pression, et qui sont agencés pour stopper la progression du front solution/gaz lorsque l'on détecte ce niveau déterminé de remplissage du réservoir 22 par la solution sanguine (par exemple 4/5ème de remplissage) et/ou une arrivée de la solution sanguine par le passage 44.

Ainsi, dans le procédé selon l'invention, on stoppe la progression du front solution/gaz dans le canal 2 après (de préférence juste après) avoir détecté ce niveau déterminé de plus fort remplissage du réservoir 22 par la solution sanguine et/ou une arrivée de la solution sanguine par le passage 44.

La progression de la solution de l'entrée 3 vers la sortie 4 du canal 2 comprend une progression d'un front entre d'une part la solution s'étendant du front vers l'entrée 3 du canal 2 et d'autre part un gaz s'étendant du front vers la sortie 4 du canal 2.

Ce front est dans la présente description appelé « front » ou « front solution/gaz »

Ainsi, le front n'étant pas un front entre deux liquides différents, il n'y a pas de risque de diffusion aléatoire d'un liquide à un autre qui pourrait poser des problèmes de reproductibilité.

Le gaz est de l'air. Dans d'autres variantes, le gaz pourrait être quelconque, de préférence :

- un gaz neutre parmi hélium, néon, argon, krypton, xénon, et radon ou un mélange de ceux-ci, et/ou
- de l'oxygène $O_2$, et/ou
- de l'azote $N_2$, et/ou
- de l'hydrogène $H_2$, et/ou
- du dioxyde de carbone.

La solution a, lors du passage du front par l'entrée 3 du canal 2, un taux de cisaillement aux parois internes du canal 2 supérieur à 10 000 s$^{-1}$.

Dans la présente description, tous les taux de cisaillement $\gamma_w$ aux parois internes du canal 2 sont supposées calculés avec la formule suivante :

$$\gamma_w = \frac{\Delta P}{2\mu L \sqrt{\dfrac{\pi}{S}}}$$

qui est une approximation de type Poiseuille avec un coefficient de perte de charge, avec

$\Delta P$ la différence de pression $P_e$-$P_s$ entre la pression $P_e$ à l'entrée 3 et la pression $P_s$ à la sortie 4 du canal 2, cette différence (ou du moins la consigne de cette différence imposée aux moyens 7) étant constante (pour mimer au plus près les conditions de circulation du sang dans le système cardiovasculaire) et typiquement comprise entre 5 et 10 kPa.

$S$ l'aire de la section interne du canal 2, que cette section soit circulaire dans un modèle type Poiseuille ou, en première approximation, carrée ou rectangulaire (de préférence avec de grands côtés du rectangle moins de deux fois plus grand que les petits côtés du rectangle) ou autre,

$\mu$ la viscosité dynamique de la solution (en Pa.s), théorique ou mesurée avec une viscosimètre de Couette

$L$ la longueur du canal 2 entre son entrée 3 et :

- le front solution/gaz si la solution n'a pas encore atteint la sortie 4, ou
- la sortie 4 si la solution a atteint la sortie 4

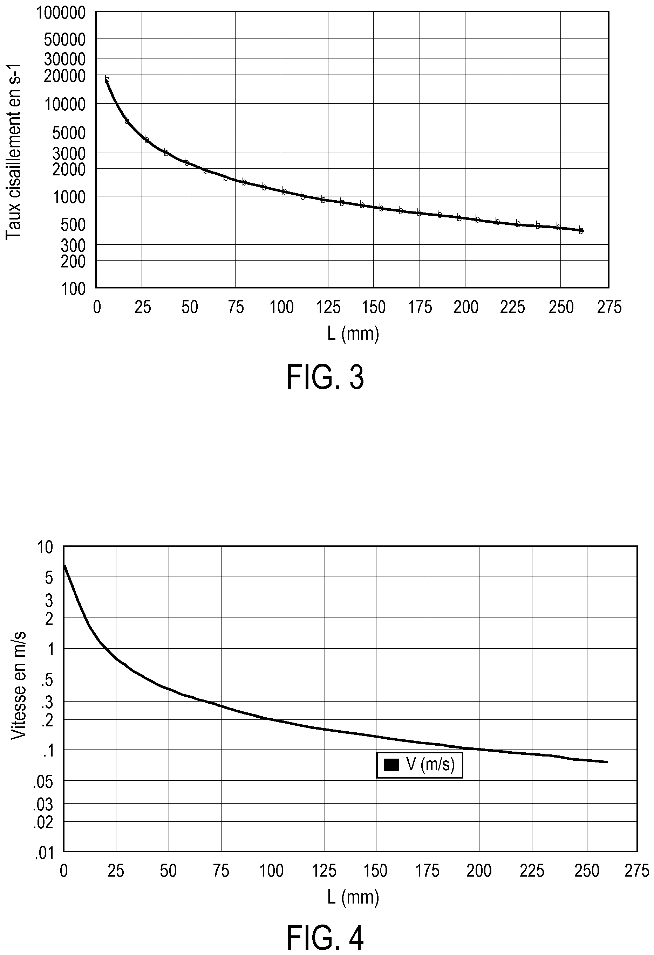

La figure 3 illustre la variation du taux de cisaillement de la solution aux parois internes du canal 2, en fonction de la distance entre l'entrée 3 du canal 2 et le front solution/gaz.

Comme illustré sur la figure 3, la solution a, pendant la progression de la solution de l'entrée 3 vers la sortie 4 du canal 2, un taux de cisaillement aux parois internes du canal qui diminue avec le temps, plus exactement pendant la progression du front de l'entrée 3 jusqu'à la sortie 4, c'est-à-dire pendant un régime transitoire qui précède un régime stationnaire.

La solution a, lors du passage du front par la sortie 4 du canal 2, un taux de cisaillement aux parois internes du canal inférieur à 1000 s$^{-1}$.

En première approximation, à un instant donné (pour une position donnée du front dans le canal 2), le taux de cisaillement de la solution aux parois internes du canal 2 est constante de l'entrée 3 du canal 2 jusqu'au front, ou jusqu'à la sortie 4 si le canal 2 est intégralement rempli par la solution.

Ainsi, l'invention donne accès à des forts taux de cisaillement (supérieur à 10 000 s$^{-1}$, sténose modérée), et à des faibles taux de cisaillement (inférieur à 1 000 s$^{-1}$, valeur physiologique « normale »). Les plaquettes sont activées par l'accélération des taux de cisaillement mais adhèrent dans une zone de décélération et forment des agrégats stables où le cisaillement est redevenu physiologique. L'invention permet donc de mimer un intervalle de conditions allant de conditions pathologiques où le gradient dégressif de cisaillement est le principal activateur des plaquettes qui adhèrent pour former le thrombus, à des conditions physiologiques pour stabiliser les agrégats plaquettaires. L'invention permet de stimuler les plaquettes par un gradient temporel dégressif de cisaillement sans ajout d'activateurs et la mesure du signal électrique sur une (ou chaque) paire 18, 19 d'électrodes recouverte d'agrégats plaquettaires est effectuée à des cisaillements physiologiques pour avoir une reproductibilité maximale.

La figure 4 illustre la variation de la vitesse du front solution/gaz, en fonction de la distance entre l'entrée 3 du canal 2 et le front solution/gaz.

Comme illustré sur la figure 4, le front a une vitesse de progression dans le canal 2 qui diminue avec le temps pendant la progression de la solution dans le canal 2, de préférence jusqu'à ce que la solution atteigne la sortie 4 du canal 2, plus exactement pendant la progression du front de l'entrée 3 jusqu'à la sortie 4, c'est-à-dire pendant le régime transitoire. Ainsi, l'invention donne accès à une variation de la vitesse d'écoulement. Ces conditions reflètent la diminution de vitesse d'écoulement du sang dans la circulation artérielle, de l'aorte vers les capillaires.

Ce mode de réalisation de procédé selon l'invention comprend un passage de la solution dans une zone de mesure du canal munie d'électrodes 8, 9 de préférence d'au moins une paire 18 ou 19 d'électrodes, ou même de plusieurs paires d'électrodes 18, 19.

Chaque paire 18 ou 19 d'électrodes est de préférence située au niveau d'une des boucles 6 du canal 2.

Les électrodes 8, 9 ont une bonne tenue à l'oxydation malgré de nombreux passages de sang, acides dilués de rinçage, sérum physiologique de rinçage, etc.

Les électrodes 8, 9 (situées à l'intérieur du canal 2), leurs pistes électroniques respectives 88, 99 et leurs connecteurs respectifs 28, 29 comprennent par exemple du platine et/ou de l'or, de préférence une couche supérieure de platine et/ou d'or en contact de la solution lors de la mesure de tension ci-après décrite.

Chaque électrode 8, 9, chaque piste 88, 99 et chaque connecteur 28, 29 est par exemple réalisé(e) par dépôt successif de trois couches de largeur 350 $\mu$m pour les électrodes, 300 $\mu$m pour les pistes et 800 $\mu$m pour les connecteurs:

- 5 $\mu$m à 8 $\mu$m d'épaisseur de cuivre, puis
- Un éventuel dopage au palladium (optionnel), puis
- 2 $\mu$m à 5 $\mu$m d'épaisseur de nickel, puis
- 0,05 à 0,1 $\mu$m d'épaisseur d'or.

Le dépôt électrolytique des électrodes 8, 9, des pistes électroniques 88, 99 et des connecteurs 28, 29 est réalisé par exemple par la technologie de structuration directe par laser (« Laser Direct Structuring » ou LDS-LPKF), par exemple par un Laser LPKF MicroLine 3D 160i piloté par le logiciel LPKF MicroLine 3D Office Software. La métallisation (Cuivre, Palladium, nickel, or) est ensuite effectuée par dépôts chimiques successifs.

Deux électrodes respectivement 8 ou 9 d'une même paire respectivement 18 ou 19 sont de préférence distantes d'un écartement de 100 $\mu$m à 4 mm, de préférence entre 300 $\mu$m et 1,5 mm, de préférence autour de 1mm.

Ce mode de réalisation de procédé selon l'invention comprend en outre une mesure, par lesdites électrodes 8, 9 et les moyens de mesure 10, d'un signal électrique alors que lesdites électrodes 8, 9 sont recouvertes par la solution dans le canal 2.

La différence de pression entre l'entrée 3 et la sortie 4 du canal 2 (ou du moins la consigne de cette différence de pression imposée aux moyens 7) est constante pendant la progression de la solution dans le canal 2, de préférence au moins jusqu'à ce que le front atteigne la sortie 4 du canal 2 et/ou jusqu'à la fin de la mesure du signal électrique par chaque paire 18 ou 19 d'électrodes.

La mesure du signal électrique est effectuée alors que :

- la progression de la solution de l'entrée 3 vers la sortie 4 du canal 2 est dans le régime stationnaire (vitesse moyenne de la solution et cisaillement de la solution aux parois internes du canal 2 constant en fonction du temps, ce régime stationnaire faisant suite au régime transitoire précédemment décrit), et
- le canal 2 est entièrement rempli par la solution de l'entrée 3 jusqu'à la sortie 4 du canal 2.

En effet, ce régime stationnaire démarre dès que le canal 2 est entièrement rempli par la solution de l'entrée 3 jusqu'à la sortie 4.

Dans ce régime stationnaire, la vitesse de la solution varie spatialement selon un profil de vitesse, plus rapide au centre de la section du canal 2 et plus lente sur les parois internes du canal 2, mais ce profil de vitesse est stable dans le temps pour n'importe quel point entre l'entrée 3 et la sortie 4.

La valeur du nombre de Reynolds (Re) à une section du canal 2 est calculable par la formule : Re=D.v.$\rho$/$\mu$ où :

D est le diamètre de cette section, et qui peut être remplacé dans la précédente formule par $2\sqrt{\dfrac{S}{\pi}}$ ou par le diamètre hydraulique Dh (Dh=4*S/peri = 4*larg*haut/(2*(larg+haut)), « peri » « larg » et « haut » étant respectivement le périmètre, la largeur et la hauteur de cette section interne du canal, $S$ étant l'aire de cette section interne du canal 2, que cette section soit circulaire dans un modèle type Poiseuille ou, en première approximation, carrée ou rectangulaire (de préférence avec de grands côtés du rectangle moins de deux fois plus grand que les petits côtés du rectangle) ou autre,

v est la vitesse moyenne du fluide s'écoulant à travers cette section

$\rho$ est la densité du fluide s'écoulant à travers cette section

$\mu$ est la viscosité dynamique (théorique ou mesurée avec une viscosimètre de Couette) du fluide s'écoulant à travers cette section

Pour une différence de pression de 5 kPa, les valeurs de Re (typiquement comprises entre 2 et 250 à 22°C pour une viscosité prise ici à 0.0038 Pas) miment celles du système artériel, des gros troncs aux artères de petits diamètres.

Les électrodes 8 de la deuxième paire 18 sont situées dans une deuxième moitié (de préférence dans le dernier tiers) du canal 2 à partir de l'entrée 3 du canal 2 jusqu'à la sortie 4 du canal 2 ; autrement dit, les électrodes 8 sont situées entre le milieu de la longueur du canal 2 et la sortie 4 du canal 2 (de préférence entre les 2/3 de la longueur du canal 2 et la sortie 4 du canal 2).

Ce mode de réalisation de procédé selon l'invention comprend en outre, à partir de la mesure du signal électrique, un calcul d'une donnée de discrimination plaquettaire et/ou de coagulation par des moyens techniques de calcul 11. Cette donnée est une donnée quantifiée, par exemple un nombre réel sur une échelle de un à dix.

Les moyens de calcul 11 comprennent uniquement des moyens techniques, typiquement des moyens électroniques (analogiques et/ou numériques), une unité centrale d'ordinateur, un processeur, et ou des moyens logiciels.

Les moyens de mesure 10 imposent (par une source de courant 12) une intensité électrique constante entre les électrodes 8, 9 de chaque paire 18, 19 et mesurent une tension entre les électrodes 8, 9 de chaque paire 18, 19.

L'intensité électrique a une forme constante en fonction du temps, typiquement une constante de 2 $\mu$A à 10 $\mu$A, de préférence autour de 5 $\mu$A. L'intensité reste constante pendant la mesure de tension, qui dure typiquement moins de 30 secondes, de préférence moins de 10 secondes, typiquement 5 secondes.

Le signal résultant pour chaque paire 18, 19 d'électrodes est une variation non linéaire de la tension mesurée en fonction du temps.

La mesure de tension pour chaque paire 18, 19 d'électrodes est synchronisée avec l'intensité électrique imposée à cette même paire 18, 19 d'électrodes de manière à mesurer la tension lorsque l'intensité est constante.

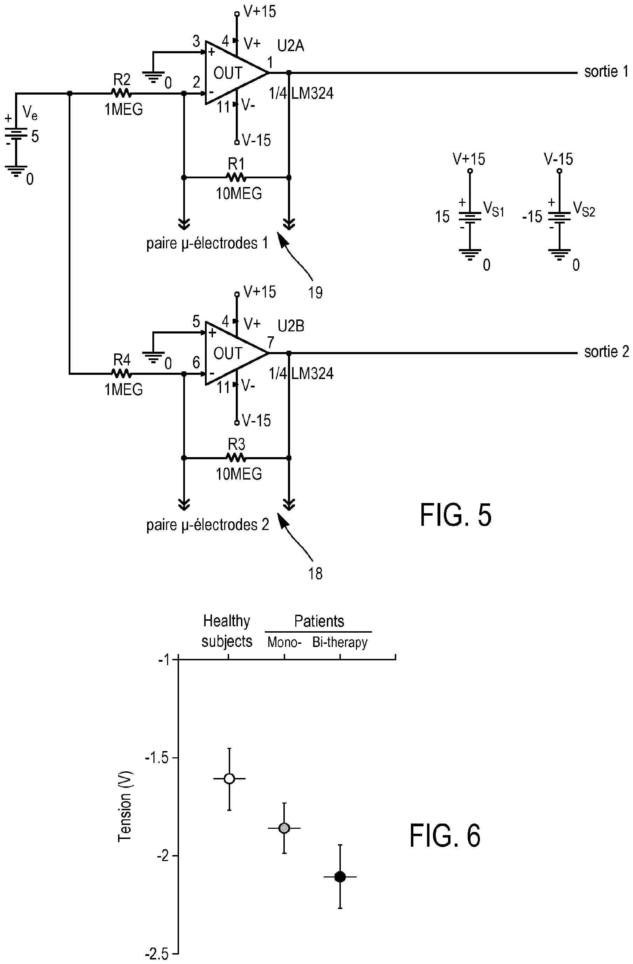

Le schéma électronique d'une partie des moyens de mesure 10, illustré sur la figure 5, est une source de courant I dont l'amplitude et la forme dépendent de la forme de la tension d'entrée $V_e$. Chaque paire 19, 18 est associée à un amplificateur opérationnel dont la tension de sortie respectivement $V_{S1}$ ou $V_{S2}$ est égale à la différence de potentiel aux bornes de la paire d'électrodes respectivement 9 ou 8. Chaque sortie $V_{S1}$ ou $V_{S2}$ de cette carte 10 est reliée à une carte d'acquisition 13.

La carte d'acquisition 13 est par exemple une carte de chez National Instruments (de référence « High-Speed M Series Multifunction DAQ for USB - 16-Bit, up to 1.25 MS/s, Integrated BNC Connectivity ») qui comporte un convertisseur analogique-numérique (CAN) 16 bits, un multiplexeur huit voies (fréquence échantillonnage maximale 1,25 MHz) et deux convertisseurs numérique-analogiques (CNA) 16 bits (2 MHz pour deux voies). Dans une variante plus compacte, la carte 13 est remplacée par un microcontrôleur.

La carte 10, le générateur 12 et la carte 13 sont programmés par les moyens de calcul 11 (ordinateur) par exemple via MatLab ou le logiciel libre Scilab.

Ce mode de réalisation de procédé selon l'invention comprend une intégration, dans le temps (typiquement sur quelques secondes, typiquement sur 2 à 20 secondes, de préférence entre t=3s et t=5 s, t=0s correspondant au début de l'imposition de l'intensité constante) de la tension mesurée pour chaque paire 18, 19 d'électrodes.

Cette intégration peut être réalisée directement par les moyens de mesure 10, ou en post-traitement par les moyens de calcul 11.

La valeur de cette intégration est directement proportionnelle à la donnée de discrimination plaquettaire en sang total, qui est une donnée dont la valeur permet de discriminer des plaquettes fonctionnant normalement, c'est-à-dire très réactives aux contraintes de cisaillement et des plaquettes ne fonctionnant pas normalement c'est-à-dire plus faiblement réactives aux contraintes de cisaillement, et permet de discriminer par exemple:

- les patients traités par antiagrégants plaquettaires des sujets sains, et/ou
- les patients traités par monothérapies antiagrégantes de ceux traités par bithérapies antiagrégantes, et/ou
- les patients traités par bithérapies antiagrégantes de ceux traités par trithérapies antiagrégantes, et/ou
- les patients traités par monothérapies antiagrégantes de ceux traités par monothérapie antiagrégante et un anticoagulant oral direct.

Ceci est illustré sur la figure 6, dans un exemple où des patients traités par monothérapies étaient traités par du prasugrel (efient) ou du clopidogrel (plavix) et des patients traités par bithérapies étaient traités par aspirine et prasugrel (efient) ou aspirine et clopidogrel (plavix).

Dans cet exemple, on a vérifié que la variation de valeur de cette intégration n'est pas un artefact de mesure ou du bruit de mesure, mais correspond bien à des variations d'agrégation plaquettaire à la surface d'électrodes. Pour cela, on a imagé par microscopie électronique à balayage (MEB), l'agrégation de plaquettes sur les électrodes 8. Après la mesure de tension sur la paire d'électrodes 8, les protéines plasmatiques et les cellules sanguines non adhérentes sont éliminées par un lavage du canal 2 avec une solution de Tyrode-Hepes-BSA (NaCl 135 mM, KCl 4 mM, MgCl2 2 mM, NaH2Po4 0.9 mM, glucose 2.5 mM, pyruvate 2.5 mM, Hépès 10 mM pH 7.35 et albumine de sérum bovin (BSA) 0.35%) pendant 1 minute. Les deux plaques de la cartouche sont dissociées et la plaque inférieure en PC Xantar 3730 LDS Noir avec les électrodes est immédiatement plongée dans le fixateur (tampon phosphate 0.1 M et glutaraldéhyde 2%). Elle y est maintenue deux heures à température ambiante puis lavée une fois par du tampon phosphate 0.2 M. Après fixation, cette plaque inférieure est conservée dans le tampon phosphate au maximum 24 h. Le matériel biologique sur cette plaque inférieure est déshydraté par des passages successifs dans des bains d'éthanol ou d'acétone de concentrations croissantes. Le matériel biologique sur cette plaque inférieure est séché par dessiccation à l'héxaméthyldisylasane. Le matériel biologique sur cette plaque inférieure est recouvert d'une fine couche d'or (20-40 nm) par pulvérisation cathodique (Scancoat Six, Edwards). La métallisation a pour but de rendre l'échantillon conducteur aux électrons évitant ainsi l'accumulation des charges parasites négatives à sa surface au cours de l'observation. Pour l'observation, le morceau de plaque métallisée est introduit dans l'enceinte sous vide secondaire du MEB (Microscope Electronique à Balayage à filament tungstène Stereoscan 260, Cambridge et système d'acquisition numérique).

L'imagerie par microscopie électronique à balayage a permis d'observer que les agrégats plaquettaires reposent sur un film fin de fibrine à la surface de toutes les électrodes.

Pour la mesure, la cartouche microfluidique 5 est connectée (du côté de connecteurs respectivement 28, 29 reliées aux pistes électroniques respectivement 88, 99 des électrodes respectivement 8, 9) :

- à la source 12 d'intensité électrique
- aux moyens de mesure 10 de la tension et
- aux moyens de calcul 11.

Les moyens 7 pour créer la différence de pression entre l'entrée 3 et la sortie 4 du canal 2 sont aussi connectés aux moyens de mesure 10 et aux moyens de calcul 11.

La carte d'acquisition 13 est connectée aux moyens de mesure 10 et aux moyens de calcul 11.

L'état morphologique de la fibrine dépend de la quantité de thrombine et des taux de cisaillement: films fin de fibrine aux taux élevés et réseaux de fibres enchevêtrées aux taux bas.

L'invention permet donc d'intégrer à la donnée calculée à partir de la mesure de tension, de l'agrégation plaquettaire et de la coagulation.

Ainsi, l'invention s'avère aussi utile pour le suivi des patients souffrant de fibrillation auriculaire traités par anticoagulants oraux en prévention du risque thromboembolique.

L'imagerie par microscopie électronique à balayage a permis de montrer une différence d'état de surface entre les électrodes 8, 9 et les parois « nues » du canal 2.

On entendra par rugosité d'une surface dans la présente description des paramètres communément notés Ra, Rz et Rt dans le domaine technique de la mesure de rugosité (dont les valeurs numériques correspondent à une mesure par un rugosimètre Mahr Perthen PGK avec unité de calcul S4P ; les mesures ont été effectuées à 23° C avec une tête de palpage MFW, un bras de palpage 250 et une longueur de palpage de 0.56 mm) dont Ra est la moyenne arithmétique des écarts (pics et creux de la hauteur de la surface) à la ligne moyenne de hauteur de la surface, Rz est la hauteur maximale définie comme la distance entre la ligne des pics et celle des creux de la hauteur de la surface, et Rt est la hauteur totale correspondant à la somme du pic maximal et du creux maximal de la hauteur de la surface.

Pour l'énergie de surface et/ou l'hydrophobicité et/ou la mouillabilité dans la présente description (mesurées à 23°C par l'intermédiaire d'un Goniomètre Digidrop GBX), les mesures d'angle de contact (d'une goutte liquide sur une surface solide en environnement gazeux) répétées pour au moins deux liquides permettent d'obtenir un système d'équation ayant pour forme générale la relation d'Owens-Wendt :

$$\frac{\gamma_L \cdot (1 + COS\theta_{SL})}{2 \cdot \sqrt{\gamma_L^D}} = \sqrt{\gamma_S^D} + \sqrt{\frac{\gamma_L^P}{\gamma_L^D}} \cdot \sqrt{\gamma_S^P}$$

Où :

$\theta_{SL}$ est l'angle de contact solide / liquide

$\gamma_L$, $\gamma_L^D$, $\gamma_L^P$ sont respectivement l'énergie totale, la composante dispersive et polaire du liquide considéré

$\gamma_S^D$, $\gamma_S^P$ sont respectivement la composante dispersive et polaire de l'énergie de surface de la surface solide.

En traçant une droite y=ax+b avec y égale à la partie gauche de la précédente équation et x égal à $\sqrt{\frac{\gamma_L^P}{\gamma_L^D}}$ (par exemple avec 3 points correspondants à de l'eau désionisée, du formamide et de l'éthylène glycol), on obtient $a=(\gamma_S^p)^{1/2}$ et $b=(\gamma_S^d)^{1/2}$ c'est-à-dire une droite dont le coefficient directeur est la racine carrée de la composante polaire de l'énergie de surface et l'ordonnée à l'origine est la racine carrée de la composante dispersive de l'énergie de surface.

Enfin, l'énergie de surface totale $\gamma_S$ (typiquement en mJ/m$^2$) est obtenue en additionnant la composante polaire $\gamma_S^P$ et dispersive $\gamma_S^D$.

Chaque électrode 8, 9 a une rugosité Ra, Rt et/ou Rz au moins 10 fois supérieure à la rugosité respectivement Ra, Rt et/ou Rz des parois internes du canal 2 « nues » (i.e. en dehors de ces électrodes 8, 9). Chaque électrode 8, 9 a une rugosité Ra supérieure à 1 $\mu$m et/ou inférieure à 100$\mu$m, et/ou Rt supérieure à 5 $\mu$m et/ou Rz supérieure à 7 $\mu$m (approchant celle de la paroi artérielle humaine).

Un tel agencement de rugosité semble au moins en partie responsable d'une amplification de l'activation plaquettaire par augmentation locale du cisaillement. Localement le taux de cisaillement est augmenté d'un facteur trois d'après un calcul éléments finis avec du sang à 22°C pour une rugosité Rz de 10$\mu$m et un taux de cisaillement de 670 s-1 dans la conduite lisse qui correspond typiquement à une électrode de mesure 8..

Pour le mode de réalisation des figures 1 à 6, des mesures de rugosité sur le PC Xantar LDS métallisé donnent des valeurs de Ra, Rz et Rt respectivement égales à 1.91±0.36, 8.55±1.56 et 11.48±2.09 $\mu$m (SD, n=12) ; pour le PC Xantar LDS non métallisé les Ra, Rz et Rt sont respectivement 0.125±0.021, 0.695±0.007 et 1.030±0.099 $\mu$m (n=2).

Chaque électrode 8, 9 est plus hydrophobe que les parois internes du canal 2 « nues » (i.e. en dehors de ces électrodes 8,9). Chaque électrode 8, 9 a une énergie de surface totale $\gamma_S$ au moins deux fois supérieure à l'énergie de surface totale $\gamma_S$ des parois internes du canal 2 « nues » (i.e. en dehors de ces électrodes 8,9). Chaque électrode 8, 9 a une énergie de surface totale $\gamma_S$ supérieure à 65 mJ/m$^2$ et/ou l'énergie de surface totale $\gamma_S$ des parois internes du canal 2 « nues » (i.e. en dehors de ces électrodes 8, 9) est inférieure à 40 mJ/m$^2$.

Un tel agencement semble en partie responsable d'une amplification de la coagulation.

Pour le mode de réalisation des figures 1 à 6, chaque électrode 8, 9 a une énergie de surface totale $\gamma_S$ d'après une mesure expérimentale sur zone électrode 8, 9 cuivre/nickel/or égale à 76,78 mJ/m$^2$

Pour le mode de réalisation des figures 1 à 6, l'énergie de surface totale $\gamma_S$ des parois internes du canal 2 « nues » d'après une mesure expérimentale égale à 25,75 mJ/m$^2$ sur PC transparent et égale à 32,99 mJ/m$^2$ sur PC noir.

Ainsi, dans ce mode de réalisation :

- la surface du PC transparent dans lequel est fabriqué (usiné ou réalisé par injection) le microcanal 2 est très hydrophobe et va adsorber les protéines plasmatiques comme le fibrinogène et activer la voie de contact de la coagulation. La faible énergie totale de surface avec une composante polaire (de moins de 25% de l'énergie de surface totale) et la faible rugosité sont par contre plutôt défavorable à l'adhésion plaquettaire.
- le PC noir non métallisé est le moins adhésif des constituants du canal 2 de la cartouche. L'adsorption des protéines plasmatiques et l'adhésion des plaquettes au fond du canal 2 sans électrode est donc la plus faible à sa surface.

- la surface du PC métallisé est hautement adhésive. Le dépôt de métal a augmenté l'énergie de surface d'un facteur supérieur à 2 et considérablement l'hydrophobicité. L'adsorption des protéines plasmatiques et l'adhésion des plaquettes est donc maximale à la surface des électrodes 8, 9.

Ce mode de réalisation de procédé selon l'invention comprend en outre une détection, de préférence par des photodiodes non illustrées (de préférence un couple de photodiodes) disposées latéralement au canal 2, du passage du front dans le canal 2 (devant les photodiodes) entre les électrodes 8 et la sortie 4 du canal 2.

La détection du passage du front par les photodiodes est envoyée aux moyens de mesure 10 pour s'assurer que les moyens de mesure 10 attendent bien que le canal 2 soit intégralement rempli et que le réservoir 22 soit partiellement rempli (au 1/10$^{ème}$ par exemple) avant de mesurer le signal électrique entre les électrodes 8 et/ou les électrodes 9.

Ce mode de réalisation de procédé selon l'invention comprend en outre :

- une mesure, de préférence par les photodiodes non illustrées (de préférence un couple de photodiodes) disposées latéralement au canal 2 (de préférence au-dessus ou au-dessous de la cartouche 1), d'une vitesse de la solution (de préférence du front) dans le canal 2 (devant les photodiodes) entre les électrodes 8 et la sortie 4 du canal 2, et
- un calcul, par des moyens techniques de traitement (par exemple par les moyens de calcul 11), d'une viscosité de la solution en fonction de la vitesse mesurée.

Il est assez difficile de mesurer une vitesse avec les photodiodes quand le canal 2 est plein.

Ainsi, on mesure de préférence la vitesse moyenne du front entre deux points, chaque point correspondant à une position d'une des photodiodes. Les points sont de préférence situés à proximité de la sortie 4 du canal 2, là où la variation de vitesse du front entre ces deux points est faible. Avec deux photodiodes espacées de 2 à 10mm par exemple on mesure la vitesse sous un ΔP constant et connu. Ceci permet de remonter à la viscosité moyenne de la solution avec la formule de Poiseuille.

L'évaluation de la viscosité sanguine est un critère supplémentaire pour évaluer le bénéfice/risque des bithérapies/trithérapies antiplaquettaires.

Ce mode de réalisation de procédé selon l'invention comprend une stabilisation en température du canal 2 et de la cartouche 5, de préférence à une température comprise entre 36°C et 38 °C, de préférence entre 36,5°C et 37,5 °C. Cela améliore la reproductibilité des mesures, car la viscosité de la solution dépend de la température. Pour cela, la cartouche 5 dans laquelle est réalisé le canal 2 est typiquement en contact d'un thermostat (intégré dans un support portant la cartouche 1, avec une résistance chauffante et/ou un module Peltier), agencé pour fonctionner en refroidissement comme en chauffage.

Bien sûr, l'invention n'est pas limitée aux exemples qui viennent d'être décrits et de nombreux aménagements peuvent être apportés à ces exemples sans sortir du cadre de l'invention.

Par exemple, dans des variantes combinables entre elles :

- le nombre de paires d'électrodes 8, 9 telles que décrites précédemment peut être quelconque, chaque paire d'électrode étant de préférence située au niveau d'une des boucles 6 du canal 2 (de préférence une seule paire d'électrode par boucle); et/ou
- le procédé est mis en oeuvre uniquement avec les électrodes 8:

  ◦ soit dans le dispositif 1,
  ◦ soit dans une variante du dispositif 1 ne comprenant pas les électrodes 9 ; c'est-à-dire dans un mode de réalisation ne comprenant qu'une seule paire d'électrodes 8, et/ou

- la mesure peut être effectuée avec les électrodes 8 alors que les électrodes 9 sont passives. De manière générale, il peut y avoir, entre l'entrée 3 et les électrodes de mesure 8, une ou plusieurs tache(s) ou dépôt(s) 9 (de préférence d'or ou de platine) dans le canal 2, chaque tache ou dépôt 9 étant agencé(e) pour être au contact de la solution sanguine remplissant le canal 2. De préférence :

  ◦ chaque paire d'électrode 8 et/ou chaque tache(s) ou dépôt(s) 9 est de préférence situé au niveau d'une des boucles 6 du canal 2 (de préférence une seule paire d'électrode 8 ou de taches ou de dépôts 9 par boucle) ; et/ou
  ◦ chaque électrode 8, 9 et/ou chaque tache ou dépôt 9 a une rugosité Ra, Rt et/ou Rz au moins 10 fois supérieure à la rugosité respectivement Ra, Rt et/ou Rz des parois internes du canal 2 « nues » (i.e. en dehors de ces électrodes 8, 9 ou taches ou dépôts 9). Un tel agencement de rugosité semble au moins en partie responsable d'une amplification de l'activation plaquettaire par augmentation locale du cisaillement.
  ◦ chaque électrode 8, 9 et/ou chaque tache ou dépôt 9 a une rugosité Ra supérieure à 1 μm et/ou inférieure à

100$\mu$m, et/ou Rt supérieure à 5 $\mu$m et/ou Rz supérieure à 7 $\mu$m (approchant celle de la paroi artérielle humaine). Un tel agencement de rugosité semble au moins en partie responsable d'une amplification de l'activation plaquettaire par augmentation locale du cisaillement.

◦ chaque électrode 8, 9 et/ou chaque tache ou dépôt 9 est plus hydrophobe que les parois internes du canal 2 « nues » (i.e. en dehors de ces électrodes ou taches ou dépôts 9). Un tel agencement semble en partie responsable d'une amplification de la coagulation

◦ chaque électrode 8, 9 et/ou chaque tache ou dépôt 9 a une énergie de surface totale $\gamma_S$ au moins deux fois supérieure à l'énergie de surface totale $\gamma_S$ des parois internes du canal 2 « nues » (i.e. en dehors de ces électrodes ou taches ou dépôts 9). Un tel agencement semble en partie responsable d'une amplification de la coagulation

◦ chaque électrode 8, 9 et/ou chaque tache ou dépôt 9 a une énergie de surface totale $\gamma_S$ supérieure à 65 mJ/m$^2$ et/ou l'énergie de surface totale $\gamma_S$ des parois internes du canal 2 « nues » (i.e. en dehors de ces électrodes ou taches ou dépôts 9) est inférieure à 40 mJ/m$^2$. Un tel agencement semble en partie responsable d'une amplification de la coagulation.

Par exemple, une variante du mode de réalisation précédemment décrit en référence aux figures comprendrait la paire d'électrodes 8, et au moins cinq paires de tache(s) ou dépôt(s) 9 d'or dans le canal (ressemblant à la paire d'électrodes 9 mais sous une forme « passive », sans piste 99) en amont des électrodes 8 (i.e. entre l'entrée 3 et les électrodes 8); et/ou

- la régulation de la pression d'aspiration est précise à moins de 0,5 kPa autour de seuil de consigne de pression fixé pour avoir une pression différentielle plus faible que les modes de réalisation précédemment décrits (typiquement de 0,2 kPa) par rapport à la pression atmosphérique du jour de la mesure, permettant d'avoir au niveau des électrodes des taux de cisaillement inférieurs (typiquement de 250 à 80s$^{-1}$) de manière à favoriser la formation de thrombus riche en fibrine et globules rouges, et ainsi ne pas se limiter au suivi des thérapies antiplaquettaires. Pour une différence de pression $\Delta P$ de 0,2 kPa, les valeurs de Re (typiquement comprises entre 0,01 et 10 à 22°C) Re approchent celles des artérioles ; et/ou
- de manière générale, la différence de pression $\Delta P$ est supérieure à 0.1 kPa (ou même supérieure à 5 kPa) et/ou inférieure à 15 kPa (ou même inférieure à 10 kPa).

Bien entendu, les différentes caractéristiques, formes, variantes et modes de réalisation de l'invention peuvent être associées les unes avec les autres selon diverses combinaisons dans la mesure où elles ne sont pas incompatibles ou exclusives les unes des autres. En particulier toutes les variantes et modes de réalisation décrits précédemment sont combinables entre eux.

## Revendications

1. Procédé de caractérisation d'un échantillon sanguin, comprenant :

   - une insertion dans un canal (2) d'une solution comprenant des plaquettes sanguines, ledit canal comprenant une entrée (3) et une sortie (4), la solution étant insérée par l'entrée du canal,
   - une création d'une différence de pression entre l'entrée et la sortie du canal, de manière à faire progresser la solution de l'entrée vers la sortie du canal,
   - un passage de la solution dans une zone de mesure du canal munie d'électrodes (8),
   - une mesure, par lesdites électrodes et des moyens de mesure (10), d'un signal électrique alors que lesdites électrodes sont recouvertes par la solution dans le canal,

   **caractérisé en ce que** la progression de la solution de l'entrée vers la sortie du canal comprend une progression d'un front entre d'une part la solution s'étendant du front vers l'entrée du canal et d'autre part un gaz s'étendant du front vers la sortie du canal
   la différence de pression entre l'entrée et la sortie du canal ou une consigne de cette différence de pression étant constante pendant la progression de la solution dans le canal.

2. Procédé selon la revendication 1, **caractérisé en ce que** la solution a, lors du passage du front par l'entrée du canal, un taux de cisaillement aux parois internes du canal supérieur à 10 000 s$^{-1}$.

3. Procédé selon la revendication 1 ou 2, **caractérisé en ce que** la solution a, pendant la progression du front de l'entrée vers la sortie du canal, un taux de cisaillement aux parois internes du canal qui diminue avec le temps.

4. Procédé selon la revendication 3, **caractérisé en ce que** la solution a, pendant la progression du front de l'entrée vers la sortie du canal, un taux de cisaillement aux parois internes du canal qui diminue avec le temps jusqu'à ce que le front atteigne la sortie du canal.

5. Procédé selon l'une quelconque des revendications précédentes, **caractérisé en ce que** la solution a, lors du passage du front par la sortie du canal, un taux de cisaillement aux parois internes du canal inférieur à $1000$ s$^{-1}$.

6. Procédé selon l'une quelconque des revendications précédentes, **caractérisé en ce que** la différence de pression entre l'entrée et la sortie du canal ou une consigne de cette différence de pression est constante pendant la progression de la solution dans le canal au moins jusqu'à ce que le front atteigne la sortie du canal et/ou jusqu'à la mesure.

7. Procédé selon l'une quelconque des revendications précédentes, **caractérisé en ce que** le canal a une section d'aire constante de son entrée à sa sortie.

8. Procédé selon l'une quelconque des revendications précédentes, **caractérisé en ce que** la différence de pression entre l'entrée et la sortie du canal est créée par aspiration du côté de la sortie du canal.

9. Procédé selon l'une quelconque des revendications précédentes, **caractérisé en ce que** le front a une vitesse de progression dans le canal qui diminue avec le temps pendant la progression de la solution dans le canal.

10. Procédé selon la revendication 9, **caractérisé en ce que** le front a une vitesse de progression dans le canal qui diminue avec le temps pendant la progression de la solution dans le canal jusqu'à ce que le front atteigne la sortie du canal.

11. Procédé selon l'une quelconque des revendications précédentes, **caractérisé en ce que** le gaz est de l'air.

12. Procédé selon l'une quelconque des revendications précédentes, **caractérisé en ce que** la mesure du signal électrique est effectuée alors que la progression de la solution de l'entrée vers la sortie du canal est dans un régime stationnaire, ledit canal étant rempli par la solution de l'entrée jusqu'à la sortie du canal.

13. Procédé selon l'une quelconque des revendications précédentes, **caractérisé en ce que** les électrodes (8) sont situées dans une deuxième moitié du canal à partir de l'entrée du canal.

14. Procédé selon l'une quelconque des revendications précédentes, **caractérisé en ce qu'**il comprend en outre, à partir de la mesure du signal électrique, un calcul d'une donnée de discrimination plaquettaire.

15. Procédé selon l'une quelconque des revendications précédentes, **caractérisé en ce que** les moyens de mesure imposent une intensité électrique constante entre les électrodes et mesurent une tension entre les électrodes pendant que l'intensité électrique est imposée.

16. Procédé selon la revendication 15, **caractérisé en ce qu'**il comprend une intégration, dans le temps, de la tension mesurée.

17. Procédé selon l'une quelconque des revendications précédentes, **caractérisé en ce qu'**il comprend :

    - une détection, de préférence par des photodiodes, du passage du front entre les électrodes et la sortie du canal, et/ou
    - une mesure, de préférence par des photodiodes, d'une vitesse de la solution dans le canal, et un calcul, par des moyens techniques de traitement, d'une viscosité de la solution en fonction de la vitesse mesurée.

18. Procédé selon l'une quelconque des revendications précédentes, **caractérisé en ce qu'**il comprend une stabilisation en température du canal, de préférence à une température comprise entre 36°C et 38 °C.

19. Procédé selon l'une quelconque des revendications précédentes, **caractérisé en ce que** la solution est un échantillon sanguin provenant d'un prélèvement sur un homme ou un animal, le procédé ne comprenant pas d'ajout d'activateur plaquettaire à l'échantillon sanguin tel que prélevé.

20. Procédé selon l'une quelconque des revendications précédentes, **caractérisé en ce que** les électrodes sont des électrodes métallisées.

**Patentansprüche**

1. Verfahren zum Charakterisieren einer Blutprobe, umfassend:

   - Einführen in einen Kanal (2) einer Lösung, umfassend Blutplättchen, der Kanal umfassend einen Einlass (3) und einen Auslass (4), wobei die Lösung durch den Einlass des Kanals eingeführt wird,
   - Erzeugen eines Druckunterschieds zwischen dem Einlass und dem Auslass des Kanals, um die Lösung von dem Einlass in Richtung des Auslasses des Kanals zu fördern,
   - Durchlaufen der Lösung einer Messzone des Kanals, die mit Elektroden (8) ausgestattet ist,
   - Messen, durch die Elektroden und Messmittel (10), eines elektrischen Signals, während die Elektroden durch die Lösung in dem Kanal bedeckt sind,

   **dadurch gekennzeichnet, dass** das Fördern der Lösung von dem Einlass in Richtung des Auslasses des Kanals ein Fördern einer Front zwischen einerseits der Lösung, die sich von der Front in Richtung des Einlasses des Kanals erstreckt, und andererseits einem Gas umfasst, das sich von der Front in Richtung des Auslasses des Kanals erstreckt,
   wobei der Druckunterschied zwischen dem Einlass und dem Auslass oder ein Sollwert dieses Druckunterschieds während des Förderns der Lösung in dem Kanal konstant ist.

2. Verfahren nach Anspruch 1, **dadurch gekennzeichnet, dass** die Lösung, bei dem Durchlaufen der Front durch den Einlass des Kanals, eine Scherrate an den Innenwänden des Kanals von mehr als 10.000 s$^{-1}$ aufweist.

3. Verfahren nach Anspruch 1 oder 2, **dadurch gekennzeichnet, dass** die Lösung, während des Förderns der Front von dem Einlass in Richtung des Auslasses des Kanals, eine Scherrate an den Innenwänden des Kanals aufweist, die mit der Zeit abnimmt.

4. Verfahren nach Anspruch 3, **dadurch gekennzeichnet, dass** die Lösung, während des Förderns der Front von dem Einlass zu dem Auslass des Kanals, eine Scherrate an den Innenwänden des Kanals aufweist, die mit der Zeit abnimmt, bis die Front den Auslass des Kanals erreicht.

5. Verfahren nach einem der vorstehenden Ansprüche,
   **dadurch gekennzeichnet, dass** die Lösung, bei dem Durchlaufen der Front durch den Auslass des Kanals, eine Scherrate an den Innenwänden des Kanals von weniger als 1.000 s$^{-1}$ aufweist.

6. Verfahren nach einem der vorstehenden Ansprüche,
   **dadurch gekennzeichnet, dass** der Druckunterschied zwischen dem Einlass und dem Auslass des Kanals oder ein Sollwert dieses Druckunterschieds während des Förderns der Lösung in dem Kanal mindestens bis die Front den Auslass des Kanals erreicht und/oder bis zu dem Messen konstant ist.

7. Verfahren nach einem der vorstehenden Ansprüche,
   **dadurch gekennzeichnet, dass** der Kanal einen konstanten Querschnitt von seinem Einlass bis zu seinem Auslass aufweist.

8. Verfahren nach einem der vorstehenden Ansprüche,
   **dadurch gekennzeichnet, dass** der Druckunterschied zwischen dem Einlass und dem Auslass des Kanals durch eine Saugwirkung von der Seite des Auslasses des Kanals erzeugt wird.

9. Verfahren nach einem der vorstehenden Ansprüche,
   **dadurch gekennzeichnet, dass** die Front eine Fördergeschwindigkeit in dem Kanal aufweist, die während des Förderns der Lösung in dem Kanal mit der Zeit abnimmt.

10. Verfahren nach Anspruch 9, **dadurch gekennzeichnet, dass** die Front eine Fördergeschwindigkeit in dem Kanal aufweist, die während des Förderns der Lösung in dem Kanal mit der Zeit abnimmt, bis die Front den Auslass des Kanals erreicht.

**11.** Verfahren nach einem der vorstehenden Ansprüche, **dadurch gekennzeichnet, dass** das Gas Luft ist.

**12.** Verfahren nach einem der vorstehenden Ansprüche, **dadurch gekennzeichnet, dass** das Messen des elektrischen Signals ausgeführt wird, während das Fördern der Lösung von dem Einlass in Richtung des Auslasses des Kanals in einem unveränderten Zustand ist, wobei der Kanal durch die Lösung von dem Einlass bis zu dem Auslass des Kanals gefüllt ist.

**13.** Verfahren nach einem der vorstehenden Ansprüche, **dadurch gekennzeichnet, dass** die Elektroden (8) in einer zweiten Hälfte des Kanals von dem Einlass des Kanals gelegen sind.

**14.** Verfahren nach einem der vorstehenden Ansprüche, **dadurch gekennzeichnet, dass** es ferner, von dem Messen des elektrischen Signals, ein Berechnen einer Plättchenunterscheidungsgröße umfasst.

**15.** Verfahren nach einem der vorstehenden Ansprüche, **dadurch gekennzeichnet, dass** die Messmittel eine konstante Stromstärke zwischen den Elektroden auferlegen und eine Spannung zwischen den Elektroden messen, während die Stromstärke auferlegt wird.

**16.** Verfahren nach Anspruch 15, **dadurch gekennzeichnet, dass** es eine zeitliche Integration der gemessenen Spannung umfasst.

**17.** Verfahren nach einem der vorstehenden Ansprüche, **dadurch gekennzeichnet, dass** es umfasst:

- Erfassen, vorzugsweise durch Fotodioden, des Durchlaufens der Front zwischen den Elektroden und dem Auslass des Kanals, und/oder
- Messen, vorzugsweise durch Fotodioden, einer Geschwindigkeit der Lösung in dem Kanal, und Berechnen, durch technische Verarbeitungsmittel, einer Viskosität der Lösung in Abhängigkeit von der gemessenen Geschwindigkeit.

**18.** Verfahren nach einem der vorstehenden Ansprüche, **dadurch gekennzeichnet, dass** es eine Temperaturstabilisierung des Kanals umfasst, vorzugsweise bei einer Temperatur zwischen 36 °C und 38 °C.

**19.** Verfahren nach einem der vorstehenden Ansprüche, **dadurch gekennzeichnet, dass** die Lösung eine Blutprobe ist, die von einer Entnahme an einem Menschen oder einem Tier stammt, wobei das Verfahren keinen Zusatz eines Plättchenaktivators zu der Blutprobe wie entnommen umfasst.

**20.** Verfahren nach einem der vorstehenden Ansprüche, **dadurch gekennzeichnet, dass** die Elektroden metallisierte Elektroden sind.

**Claims**

**1.** Process for characterizing a blood sample, comprising:

- introducing a solution comprising blood platelets into a channel (2), said channel comprising an inlet (3) and an outlet (4), the solution being introduced through the inlet of the channel,
- creating a pressure difference between the inlet and the outlet of the channel, so as to move the solution forward from the inlet towards the outlet of the channel,
- passing the solution into a measurement zone of the channel equipped with electrodes (8),
- measuring an electrical signal using measurement means (10) and said electrodes, while said electrodes are covered with the solution in the channel,
**characterized in that** the forward movement of the solution from the inlet towards the outlet of the channel comprises a forward movement of a front between

- the solution extending from the front towards the inlet of the channel on the one hand, and
- a gas extending from the front towards the outlet of the channel on the other hand

the pressure difference between the inlet and the outlet of the channel or a set-point of this pressure difference being constant during the forward movement of the solution in the channel.

2. Process according to claim 1, **characterized in that**, during the passage of the front through the inlet of the channel, the solution has a shear rate at the internal walls of the channel greater than 10,000 s$^{-1}$.

3. Process according to claim 1 or 2, **characterized in that**, during the forward movement of the front from the inlet towards the outlet of the channel, the solution has a shear rate at the internal walls of the channel which decreases with time.

4. Process according to claim 3, **characterized in that**, during the forward movement of the front from the inlet towards the outlet of the channel, the solution has a shear rate at the internal walls of the channel which decreases with time until the front reaches the outlet of the channel.

5. Process according to any one of the preceding claims, **characterized in that**, during the passage of the front through the outlet of the channel, the solution has a shear rate at the internal walls of the channel less than 1000 s$^{-1}$.

6. Process according to any one of the preceding claims, **characterized in that** the pressure difference between the inlet and the outlet of the channel or a set-point of this pressure difference is constant during the forward movement of the solution in the channel, preferably at least until the front reaches the outlet of the channel and/or up to the measurement.

7. Process according to any one of the preceding claims, **characterized in that** the channel has a cross-sectional area that is constant from its inlet to its outlet.

8. Process according to any one of the preceding claims, **characterized in that** the pressure difference between the inlet and the outlet of the channel is created by suction on the side with the outlet of the channel.

9. Process according to any one of the preceding claims, **characterized in that** the front has a forward movement velocity in the channel which decreases with time during the forward movement of the solution in the channel.

10. Process according to claim 9, **characterized in that** the front has a forward movement velocity in the channel which decreases with time during the forward movement of the solution in the channel until the front reaches the outlet of the channel.

11. Process according to any one of the preceding claims, **characterized in that** the gas is air.

12. Process according to any one of the preceding claims, **characterized in that** the measurement of the electrical signal is carried out when the progression of the solution from the inlet towards the outlet of the channel is in a steady state, said channel being filled with the solution from the inlet to the outlet of the channel.

13. Process according to any one of the preceding claims, **characterized in that** the electrodes (8) are situated in a second half of the channel starting from the inlet of the channel.

14. Process according to any one of the preceding claims, **characterized in that** it also comprises, based on the measurement of the electrical signal, a calculation of an item of data relating to platelet discrimination.

15. Process according to any one of the preceding claims, **characterized in that** the measurement means impose a constant electrical current between the electrodes and measure a voltage between the electrodes while the electrical current is imposed.

16. Process according to claim 15, **characterized in that** it comprises an integration, over time, of the voltage measured.

17. Process according to any one of the preceding claims, **characterized in that** it comprises:

   - detecting the passage of the front between the electrodes and the outlet of the channel, preferably by means of photodiodes, and/or

- measuring a velocity of the solution in the channel, preferably by means of photodiodes, and calculating a viscosity of the solution as a function of the velocity measured, using technical processing means.

18. Process according to any one of the preceding claims, **characterized in that** it comprises a temperature stabilization of the channel, preferably at a temperature comprised between 36°C and 38 °C.

19. Process according to any one of the preceding claims, **characterized in that** the solution is a blood sample coming from a human or an animal, the process not comprising an addition of platelet activator to the blood sample as taken.

20. Process according to any one of the preceding claims, **characterized in that** the electrodes are metallized electrodes.

FIG. 1

FIG. 2

FIG. 3

FIG. 4

FIG. 5

FIG. 6

**EP 3 535 582 B1**

## RÉFÉRENCES CITÉES DANS LA DESCRIPTION

### Documents brevets cités dans la description

- WO 2009053841 A